# EUROPÄISCHE PATENTSCHRIFT

(11) **EP 2 550 911 B1**
(45) Veröffentlichungstag und Bekanntmachung des Hinweises auf die Patenterteilung: **31.08.2016**
(21) Anmeldenummer: 12174990.7
(22) Anmeldetag: 04.07.2012
(51) Int. Cl.: A61B 1/07, A61B 1/06, A61B 1/00, G02B 3/14, G02B 23/24, G02B 26/00

(54) **Endoskop mit einstellbarer Blickrichtung**
Endoscope with adjustable view angle
Endoscope à direction d'observation réglable

(30) Priorität: 28.07.2011 DE 102011079958
(43) Veröffentlichungstag der Anmeldung: 30.01.2013
(73) Patentinhaber: Karl Storz GmbH & Co. KG, 78532 Tuttlingen (DE)
(72) Erfinder: Dr. Lei, Fang, 78532 Tuttlingen (DE)

(56) Entgegenhaltungen:
- WO-A1-2004/051323
- WO-A2-2007/033326
- US-A1- 2009 021 818
- US-A1- 2009 177 033
- US-B1- 6 261 226

## Beschreibung

Die vorliegende Erfindung bezieht sich auf ein Endoskop mit einstellbarer Blickrichtung.

Neben Endoskopen für medizinische und nicht-medizinische technische Anwendungen, deren Blickrichtung parallel zur Längsachse des Schafts des Endoskops ist, wurden bereits früh Endoskope mit anderen feststehenden Blickrichtungen entwickelt. Mit der Blickrichtung eines Endoskops ist hier und im Folgenden immer die Richtung vom distalen Ende des Endoskops aus gemeint, in der ein Gegenstand liegt, der in der Mitte des mittels des Endoskops erfassten Bilds erscheint. Bei vielen Anwendungen ist jedoch eine feste Blickrichtung nachteilig. Im ungünstigsten Fall muss beispielsweise während eines medizinischen Eingriffs mehrfach das Endoskop gewechselt werden. In solchen Fällen ist die Verwendung eines Endoskops mit einer in situ einstellbaren bzw. verstellbaren Blickrichtung vorteilhaft.

Die Beobachtung eines Gegenstands in einem Hohlraum mittels eines Endoskops setzt in der Regel eine Beleuchtung des Gegenstands voraus. Dazu weist ein Endoskop beispielsweise Lichtwellenleiter, insbesondere Glasfasern, auf, mittels derer Beleuchtungslicht vom proximalen Ende des Endoskops entlang des Schafts zum distalen Ende des Endoskops übertragen wird. Lichtaustrittsflächen der Lichtwellenleiter am distalen Ende des Endoskops sind so angeordnet und ausgebildet, dass das gesamte Sichtfeld bzw. Blickfeld ausreichend und homogen ausgeleuchtet wird.

Bei einem Endoskop mit einstellbarer Blickrichtung wird das Beleuchtungslicht am distalen Ende des Endoskops im einfachsten Fall so verteilt, dass unabhängig von der jeweils eingestellten Blickrichtung das gesamte Sichtfeld ausgeleuchtet ist. Dies hat jedoch eine Reihe von Nachteilen zur Folge. Insbesondere wird Lichtleistung verschwendet, weil ständig unabhängig von der tatsächlich eingestellten Blickrichtung die gesamten Sichtfelder aller einstellbaren Blickrichtungen ausgeleuchtet werden. Bei einer vorbestimmten erwünschten Helligkeit muss somit insgesamt eine deutlich höhere Lichtleistung zur Verfügung gestellt werden als bei einem Endoskop mit feststehender Blickrichtung.

Ein weiterer Nachteil rührt daher, dass Beleuchtungslicht hoher Intensität Gewebe oder andere Gegenstände photothermisch oder photochemisch schädigen kann. Bei einem Endoskop mit feststehender Blickrichtung fällt ein zu geringer Abstand des distalen Endes des Endoskops zu einem Gegenstand zumindest bei Betrachtung des erfassten Bilds in der Regel auf. Bei Verwendung einer Kamera am Endoskop ist auch eine automatische Warnung von Benutzern möglich, wenn die Helligkeit eines erfassten Bilds eine vorbestimmte Schwelle überschreitet. Bei einem Endoskop mit einstellbarer Blickrichtung fällt jedoch ein Teil des Beleuchtungslichts auf Gegenstände, die außerhalb des Sichtfelds liegen. Eine unerwünschte Annäherung des distalen Endes des Endoskops an diese Gegenstände und eine resultierende Bestrahlung dieser Gegenstände mit einer zu hohen Strahlungsleistung fallen deshalb nicht auf.

Ein weiterer Nachteil besteht darin, dass auch Beleuchtungslicht, das außerhalb des Sichtfelds abgestrahlt wird, von Gegenständen oder opaken Medien gestreut oder reflektiert werden kann. Das reflektierte oder gestreute Beleuchtungslicht kann direkt oder indirekt in den Beobachtungsstrahlengang gelangen. Dadurch können Kontraste und vor allem in dunklen Bildbereichen die Unterscheidbarkeit von Gegenständen verringert werden.

Ein weiterer Nachteil rührt daher, dass die Beleuchtungsstärke bzw. die Intensität des Beleuchtungslichts in der Richtung, in der die Blickrichtung variiert werden kann (oft auch als vertikale Richtung bezeichnet) im Wesentlichen konstant ist, während sie in der dazu senkrechten Richtung (oft auch als horizontale Richtung bezeichnet) in der Regel zum Rand des Sichtfelds hin leicht abnimmt. Von Endoskopen mit feststehender Blickrichtung sind Benutzer jedoch in der Regel eine Beleuchtungsstärke gewohnt, die sowohl in horizontaler als auch in vertikaler Richtung zum Rand des Sichtfelds hin leicht abnimmt. Die in vertikaler Richtung konstante Beleuchtungsstärke kann deshalb als irritierend empfunden werden.

In der DE 600 15 375 T2 ist eine Anordnung mehrerer Prismen beschrieben. Eines der Prismen ist um eine Achse rotierbar, um Beleuchtungslicht in eine einstellbare Blickrichtung zu werfen. Die Prismen erfordern jedoch einen Bauraum, der nicht in jedem Endoskop zur Verfügung steht. Ferner kann es in der Praxis aufwändig sein, mit den in der DE 600 15 375 T2 beschriebenen Anordnungen gleichzeitig eine weitgehende optische Isolation von Beleuchtungs- und Beobachtungsstrahlengang, eine hohe Lichtstärke im Beobachtungsstrahlengang, geringe Verluste im Beleuchtungsstrahlengang und einen kleinen Schaftdurchmesser zu realisieren.

Eine Aufgabe der vorliegenden Erfindung besteht darin, ein verbessertes Endoskop mit einstellbarer Blickrichtung zu schaffen.

Diese Aufgabe wird durch die Gegenstände der unabhängigen Ansprüche gelöst.

Weiterbildungen sind in den abhängigen Ansprüchen angegeben.

Ein Endoskop umfasst eine Lichtaustrittsfläche am distalen Ende des Endoskops und eine steuerbare Lichtbrechungseinrichtung zum steuerbaren Brechen von Beleuchtungslicht mittels einer formbaren Phasengrenzfläche zwischen zwei Fluiden, wobei die steuerbare Lichtbrechungseinrichtung am distalen Ende des Endoskops angeordnet ist.

Das Endoskop ist insbesondere ein Endoskop für medizinische oder technische Anwendungen, im zweiten Fall auch als Boroskop bezeichnet. Die Lichtbrechungseinrichtung umfasst insbesondere in einem hermetisch abgeschlossenen Hohlraum zwei Flüssigkeiten oder eine Flüssigkeit und ein Gas, die nicht oder nur in geringem Maß mischbar sind und deshalb eine definierte Phasengrenzfläche mit einer positiven Grenzflächenspannung bzw. Grenzflächenenergie bilden. Beispielsweise umfasst die Lichtbrechungseinrichtung Waser oder eine Salzlösung oder eine andere hydrophile bzw. lipophobe Flüssigkeit und ein Öl oder eine andere lipophile bzw. hydrophobe Flüssigkeit.

Die beiden Fluide sind insbesondere für das vorgesehene Beleuchtungslicht transparent und weisen unterschiedliche Brechungsindizes auf. Aufgrund der unterschiedlichen Brechungsindizes wird Beleuchtungslicht an der Phasengrenzfläche zwischen den beiden Fluiden gemäß dem selliusschen Brechungsgesetz gebrochen. Die Brechung des Beleuchtungslichts ist von der Form bzw. Gestalt der Phasengrenzfläche abhängig, insbesondere von der Richtung der Flächennormalen und von der Krümmung der Phasengrenzfläche.

Die Lichtbrechungseinrichtung kann ausgebildet sein, um die Phasengrenzfläche in im Wesentlichen ebener Gestalt um eine oder mehrere Achsen zu kippen. Dies bedeutet, dass zu einem gegebenen Zeitpunkt alle Flächennormalen der Phasengrenzfläche gleich oder im Wesentlichen gleich sind und gemeinsam um eine oder zwei Achsen geschwenkt werden können. Alternativ oder zusätzlich kann die Krümmung der Phasengrenzfläche verändert werden. Insbesondere weist die Phasengrenzfläche in jedem Moment überwiegend, weitgehend oder vollständig eine positive Gaußsche Krümmung K = k₁ x k₂ auf, wobei die beiden Hauptkrümmungen k₁, k₂ gleichzeitig bzw. synchron oder unabhängig voneinander veränderbar sind.

Die steuerbare Lichtbrechungseinrichtung ist insbesondere unmittelbar lichtstromaufwärts der Lichtaustrittsfläche am distalen Ende des Endoskops angeordnet. Die steuerbare Lichtbrechungseinrichtung ist somit insbesondere unmittelbar unter der bzw. angrenzend an die äußere Oberfläche des Endoskops angeordnet.

Die steuerbare Lichtbrechungseinrichtung am distalen Ende des Endoskops ermöglicht ein Lenken von Beleuchtungslicht in eine einstellbare Richtung und/oder in einen Raumwinkel bzw. Raumbereich einstellbarer Größe und Gestalt. Die steuerbare Lichtbrechungseinrichtung am distalen Ende des Endoskops ermöglicht eine Anpassung der Beleuchtung an eine einstellbare Blickrichtung und/oder an eine einstellbare Brennweite eines Objektivs im Beobachtungsstrahlengang des Endoskops. Damit können einige der oben beschriebenen Nachteile einer von einer einstellbaren Blickrichtung und/oder einer einstellbaren Größe des Sichtfelds unabhängigen Beleuchtung vermieden werden.

Insbesondere kann die Lichtleistung, die auf Gegenstände außerhalb des momentanen Sichtfelds fällt, und damit die photothermische oder photochemische Schädigung dieser Gegenstände reduziert werden. Ferner können die Reflexion oder Streuung von Beleuchtungslicht an nicht beobachteten Gegenständen vermindert und damit die Bildqualität verbessert werden. Ferner kann die Verteilung von Beleuchtungslicht an die Sehgewohnheiten der Benutzer angepasst werden, die in der Regel an eine maximale Beleuchtungsstärke bzw. Intensität in der Bildmitte und eine von der Mitte ausgehend nach allen Seiten hin abfallende Beleuchtungsstärke bzw. Intensität gewohnt sind. Ferner können bei einer gegebenen Beleuchtungslichtquelle Gegenstände innerhalb des momentanen Sichtfelds heller beleuchtet oder bei gleich heller Beleuchtung des momentanen Sichtfelds eine schwächere Beleuchtungslichtquelle gewählt werden.

Nachfolgend beschriebene Ausführungsbeispiele ermöglichen ferner eine weitgehende Miniaturisierung der Lichtbrechungseinrichtung. Die Verwendung der steuerbaren Lichtbrechungseinrichtung am distalen Ende des Endoskops steht deshalb der zunehmenden Miniaturisierung von Endoskopen nicht im Wege.

Bei einem Endoskop, wie es hier beschrieben ist, ist die Lichtaustrittsfläche am distalen Ende des Endoskops eine Lichtaustrittsfläche der steuerbaren Lichtbrechungseinrichtung.

Wenn die Lichtaustrittsfläche der steuerbaren Lichtbrechungseinrichtung gleichzeitig die Lichtaustrittsfläche des Endoskops an dessen distalem Ende ist, können Reflexionen an weiteren Grenzflächen vermieden und der erforderliche Bauraum weiter reduziert werden.

Ein Endoskop, wie es hier beschrieben ist, kann eine Mehrzahl von Lichtaustrittsflächen am distalen Ende aufweisen, wobei an jeder der Mehrzahl von Lichtaustrittsflächen eine Lichtbrechungseinrichtung angeordnet ist.

Jede Lichtbrechungseinrichtung ist insbesondere unmittelbar lichtstromaufwärts der zugeordneten Lichtaustrittsfläche angeordnet, wobei die zugeordnete Lichtaustrittsfläche des Endoskops jeweils mit der Lichtaustrittsfläche der Lichtbrechungseinrichtung identisch sein kann. Eine Mehrzahl von Lichtaustrittsflächen ermöglicht beispielsweise eine schattenfreie oder schattenarme Beleuchtung von Gegenständen, wenn die Lichtaustrittsflächen an gegenüberliegenden Seiten einer Lichteintrittsfläche in einem Beobachtungsstrahlengang angeordnet sind. Ferner kann eine Mehrzahl von Lichtaustrittsflächen mit einer Mehrzahl von Lichtbrechungseinrichtungen eine Vergrößerung des beleuchtbaren Raumbereichs oder eine Konzentration des zur Verfügung stehenden Lichtes auf einen gegebenen Raumbereichermöglichen.

Neben der Mehrzahl von Lichtaustrittsflächen, denen jeweils eine Lichtbrechungseinrichtung zugeordnet ist, kann das Endoskop eine oder mehrere weitere Lichtaustrittsflächen ohne zugeordnete Lichtbrechungseinrichtung aufweisen.

Bei einem Endoskop, wie es hier beschrieben ist, ist die steuerbare Lichtbrechungseinrichtung insbesondere zumindest entweder zum Einstellen der Beleuchtungsrichtung oder zum Einstellen des Öffnungswinkels des zu beleuchtenden Raumbereichs ausgebildet.

Bei einem Endoskop, wie es hier beschrieben ist, kann die Lichtbrechungseinrichtung einen Querschnitt aufweisen, bei dem zumindest entweder dessen Fläche oder dessen Breite in der vorgesehenen Ausbreitungsrichtung von Beleuchtungslicht zunimmt.

Alternativ kann bei einem Endoskop, wie es hier beschrieben ist, die Lichtbrechungseinrichtung einen Querschnitt aufweisen, bei dem zumindest entweder dessen Fläche oder dessen
Breite in der vorgesehenen Ausbreitungsrichtung von Beleuchtungslicht abnimmt. Mit dem Querschnitt ist ein Querschnitt senkrecht oder im Wesentlichen senkrecht zur vorgesehenen Lichtstromrichtung oder mittleren Lichtstromrichtung innerhalb der Lichtbrechungseinrichtung gemeint. Die Lichtstromrichtung oder die mittlere Lichtstromrichtung ist insbesondere die Richtung von einer Lichteintrittsfläche zu einer Lichtaustrittsfläche der Lichtbrechungseinrichtung. Durch einen in der vorgesehenen Lichtstromrichtung variierenden Querschnitt der Lichtbrechungseinrichtung kann die Divergenz von Beleuchtungslicht verändert werden. Beispielsweise kann durch einen in Lichtstromrichtung schmäler werdenden Querschnitt die Divergenz von Beleuchtungslicht erhöht oder durch einen in Lichtstromrichtung breiter werdenden Querschnitt die Divergenz von Beleuchtungslicht reduziert werden. Somit kann die Lichtbrechungseinrichtung einen Beitrag zur Anpassung der Verteilung des Beleuchtungslichts an die Größe des zu beleuchtenden Raumbereichs leisten.

Ein Endoskop, wie es hier beschrieben ist, kann ferner eine Felderzeugungseinrichtung zum Erzeugen eines elektrischen oder magnetischen Felds zum Formen der Phasengrenzfläche aufweisen.

Als Felderzeugungseinrichtung kommen insbesondere Elektroden zum Erzeugen eines elektrostatischen Felds oder eines elektrischen Wechselfelds oder eine oder mehrere Spulen zum Erzeugen eines statischen oder zeitabhängigen magnetischen Felds in Betracht. Ein von einer Felderzeugungseinrichtung erzeugtes elektrisches Feld beeinflusst die Form bzw. Gestalt der Phasengrenzflächen insbesondere, wenn die beiden Fluide unterschiedliche Permittivitäten bzw. Dielektrizitätszahlen aufweisen. Ein von der Felderzeugungseinrichtung erzeugtes magnetisches Feld beeinflusst die Form der Phasengrenzfläche insbesondere, wenn die beiden Fluide unterschiedliche magnetische Suszeptibilitäten bzw. unterschiedliche magnetische Permeabilitäten aufweisen. Spulen und vor allem Elektroden können weitgehend miniaturisiert werden, so dass sie einen geringen Bauraum einnehmen und eine Miniaturisierung der Lichtbrechungseinrichtung und des Endoskops ermöglichen.

Ein Endoskop, wie es hier beschrieben ist, bei dem zumindest entweder die Blickrichtung oder der Bildfeldwinkel einstellbar ist, umfasst insbesondere eine Steuerungseinrichtung zum Erzeugen eines Steuersignals zum Steuern der Lichtbrechungseinrichtung, wobei die Steuerungseinrichtung ausgebildet ist, um die Lichtbrechungseinrichtung zumindest entweder abhängig von der eingestellten Blickrichtung oder abhängig von dem eingestellten Bildfeldwinkel zu steuern.

Das Steuersignal ist insbesondere ein elektrisches Spannungssignal, das unmittelbar an Elektroden der Lichtbrechungseinrichtung angelegt werden kann, oder ein elektrisches Stromsignal, das unmittelbar an eine Spule der Lichtbrechungseinrichtung angelegt werden kann.

Das Endoskop und die Steuerungseinrichtung des Endoskops sind insbesondere so ausgebildet, dass Benutzer an einer Benutzerschnittstelle des Endoskops die Blickrichtung einstellen können, wobei die Steuerungseinrichtung selbsttätig die Lichtbrechungseinrichtung so steuert, dass Beleuchtungslicht in eine der Blickrichtung entsprechende Richtung gelenkt wird. Alternativ oder zusätzlich sind das Endoskop und die Steuerungseinrichtung so ausgebildet, dass Benutzer an der Benutzerschnittstelle des Endoskops den Bildfeldwinkel bzw. den Raumwinkel, innerhalb dessen gleichzeitig erfasste Gegenstände liegen, einstellen können, wobei die Steuerungseinrichtung selbsttätig die Lichtbrechungseinrichtung so steuert, dass der Raumwinkelbereich, in dem Beleuchtungslicht abgestrahlt wird, dem Bildfeldwinkel entspricht. Dies kann eine einfache und effiziente Bedienung bzw. Verwendung des Endoskops ermöglichen.

Bei einem Endoskop, wie es hier beschrieben ist, mit einer Steuerungseinrichtung, kann diese ausgebildet sein, um für mehrere Lichtbrechungseinrichtungen je ein Steuersignal zu erzeugen.

Insbesondere ist die Steuerungseinrichtung mittels mehrerer Signalleitungen mit je einer von einer Mehrzahl von Lichtbrechungseinrichtungen gekoppelt, um die Lichtbrechungseinrichtungen unabhängig voneinander zu steuern und damit eine noch bessere Anpassung des beleuchtenden Bereichs an den beobachteten Bereich zu ermöglichen.

Ein Endoskop, wie es hier beschrieben ist, kann ferner einen Lichtleiter zum Übertragen von Beleuchtungslicht zum distalen Ende des Endoskops mit einer Lichtaustrittsfläche, die am distalen Ende des Endoskops angeordnet ist, umfassen, wobei die Lichtbrechungseinrichtung zwischen der Lichtaustrittsfläche des Lichtleiters und der Lichtaustrittsfläche des Endoskops angeordnet ist.

Der Lichtleiter ist insbesondere zum Übertragen von Beleuchtungslicht vom proximalen Ende des Endoskops zum distalen Ende des Endoskops ausgebildet. Am proximalen Ende des Endoskops kann eine Lichtquelle vorgesehen sein. Alternativ kann das proximale Ende des Endoskops über ein Lichtleitkabel mit einer separaten Lichtquelle zum Bereitstellen von Beleuchtungslicht gekoppelt sein. Alternativ kann eine Leuchtdiode oder eine andere miniaturisierbare Lichtquelle am distalen Ende des Endoskops unmittelbar lichtstromaufwärts der Lichtbrechungseinrichtung oder mit dieser über einen kurzen Lichtleiter gekoppelt angeordnet sein.

Insbesondere die Kombination eines Lichtleiters, der sich vom proximalen bis zum distalen Ende des Endoskops erstreckt, und einer Lichtbrechungseinrichtung am distalen Ende des Endoskops kann einen geringen Querschnitt des Schafts des Endoskops und gleichzeitig eine Steuerung des am distalen Ende des Endoskops austretenden Beleuchtungslichts mit den oben genannten Vorteilen ermöglichen.

Ein Endoskop, wie es hier beschrieben ist, kann eine Mehrzahl von Lichtleitern zum Übertragen von Beleuchtungslicht zum distalen Ende des Endoskops mit je einer Austrittsfläche, die am distalen Ende des Endoskop angeordnet ist, und eine Lichtweicheneinrichtung zum steuerbaren Einkoppeln von Beleuchtungslicht in einen oder mehrere der Mehrzahl von Lichtleitern aufweisen.

Die Lichtweicheneinrichtung und die Lichteintrittsflächen der Lichtleiter sind insbesondere am proximalen Ende des Endoskops angeordnet, wo mehr Bauraum zur Verfügung stehen kann als im Schaft des Endoskops. Einer, mehreren oder allen Lichtaustrittsflächen der Mehrzahl von Lichtleitern ist jeweils eine Lichtbrechungseinrichtung zugeordnet. Insbesondere ist jede Lichtaustrittsfläche eines Lichtleiters unmittelbar mit einer Lichteintrittsfläche einer Lichtbrechungseinrichtung gekoppelt, deren Lichtaustrittsfläche gleichzeitig Lichtaustrittsfläche des Endoskops ist. Die Kombination einer Lichtweicheneinrichtung am proximalen Ende des Endoskops mit einer oder mehreren Lichtbrechungseinrichtungen am distalen Ende des Endoskops kann bei einer geringen Anzahl von Lichtleitern und von Lichtbrechungseinrichtungen eine Anpassung der Beleuchtungsrichtung an die Blickrichtung ermöglichen.

Ein Endoskop umfasst eine Mehrzahl von Lichtaustrittsflächen am distalen Ende des Endoskops und eine Mehrzahl von steuerbaren Lichtlenkeinrichtungen zum steuerbaren Lenken von Beleuchtungslicht, wobei an jeder Lichtaustrittsfläche des Endoskops je eine der Mehrzahl von steuerbaren Lichtlenkeinrichtungen angeordnet ist.

Jede Lichtlenkeinrichtung ist insbesondere unmittelbar lichtstromaufwärts der zugeordneten Lichtaustrittsfläche des Endoskops angeordnet, wobei die Lichtaustrittsfläche des Endoskops mit der Lichtaustrittsfläche der Lichtlenkeinrichtung identisch sein kann. Jede Lichtlenkeinrichtung kann eine Lichtbrechungseinrichtung sein, wie sie hier beschrieben ist.

Bei einem Verfahren zum Beleuchten eines einstellbaren Sichtfelds eines Endoskops werden Beleuchtungslicht bereitgestellt und eine Phasengrenzfläche zwischen zwei Fluiden am distalen Ende des Endoskops zum steuerbaren Brechen von Beleuchtungslicht geformt, wobei die Phasengrenzfläche abhängig von dem momentan eingestellten Sichtfeld des Endoskops geformt wird.

Das Sichtfeld des Endoskops ist insbesondere hinsichtlich der Blickrichtung und/oder hinsichtlich des Bildfeldwinkels bzw. des Raumwinkels bezogen auf das distale Ende des Endoskops, innerhalb dessen gleichzeitig erfasste Gegenstände liegen, einstellbar. Die Phasengrenzfläche zwischen den beiden Fluiden ist insbesondere unmittelbar lichtstromaufwärts einer Lichtaustrittsfläche des Endoskops angeordnet. Das Endoskop kann ein Endoskop sein, wie es hier beschrieben ist, oder eine oder mehrere von dessen Eigenschaften und Merkmalen aufweisen.

Bei einem Verfahren, wie es hier beschrieben ist, können ferner ein Steuersignal erzeugt und das Steuersignal zum distalen Ende des Endoskops übertragen werden, wobei das Formen der Phasengrenzfläche am distalen Ende des Endoskops mittels des Steuersignals gesteuert wird.

Bei einem Verfahren, wie es hier beschrieben ist, können mehrere Steuersignale erzeugt und zum distalen Ende des Endoskops übertragen werden, um zum Brechen von Beleuchtungslicht das Formen mehrerer Phasengrenzflächen am distalen Ende des Endoskops durch je ein Steuersignal zu steuern.

Bei einem Endoskop, wie es hier beschrieben ist, kann die Lichtweicheneinrichtung zwei nicht mischbare Flüssigkeiten mit unterschiedlichen Brechungsindizes und eine Grenzflächensteuereinrichtung zum elektrischen oder magnetischen Verändern zumindest entweder der Anordnung oder der Form einer Grenzfläche zwischen den nicht mischbaren Flüssigkeiten umfassen.

### Kurzbeschreibung der Figuren

Nachfolgend werden Ausführungsformen anhand der beigefügten Figuren näher erläutert. Es zeigen:
- Figur 1: eine schematische Darstellung eines Endoskops mit einstellbarer Blickrichtung;
- Figur 2: eine schematische Darstellung einer Lichtbrechungseinrichtung;
- Figur 3: eine weitere schematische Darstellung der Lichtbrechungseinrichtung aus Figur 2;
- Figur 4: eine weitere schematische Darstellung der Lichtbrechungseinrichtung aus den Figuren 2 und 3;
- Figur 5: eine weitere schematische Darstellung der Lichtbrechungseinrichtung aus den Figuren 2 bis 4;
- Figur 6: eine schematische Darstellung einer weiteren Lichtbrechungseinrichtung;
- Figur 7: eine schematische Darstellung einer weiteren Lichtbrechungseinrichtung;
- Figur 8: ein schematisches Flussdiagramm eines Verfahrens zum Beleuchten.

### Beschreibung der Ausführungsformen

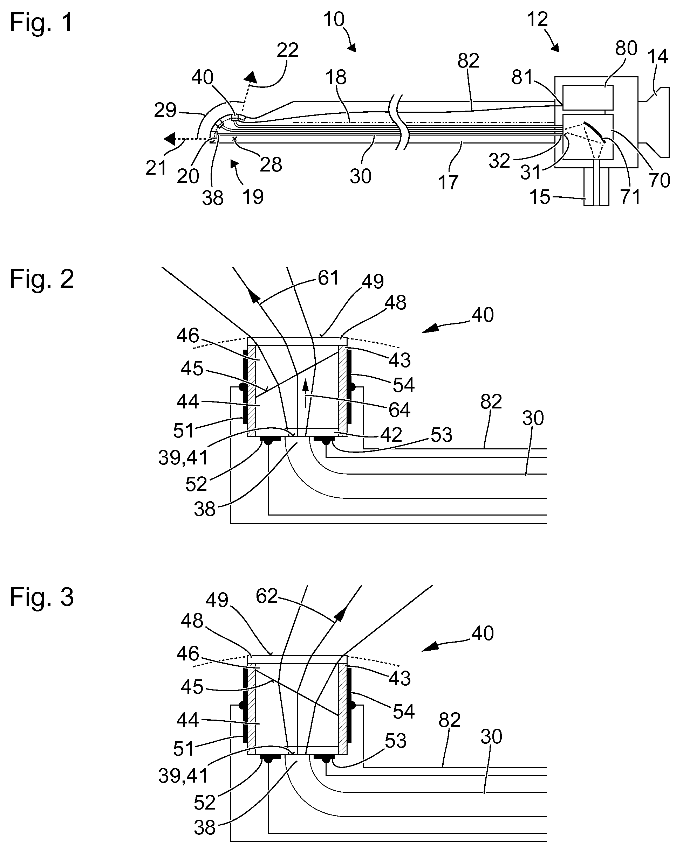

Figur 1 zeigt eine schematische Darstellung eines Endoskops 10 mit einem proximalen Ende 12, einem distalen Ende 19 und einem starren Schaft 17, der sich vom proximalen Ende 12 bis zum distalen Ende 19 erstreckt. Alternativ ist der Schaft 17 flexibel oder teilweise flexibel. Der Querschnitt des Schafts 17 oder zumindest die äußere Kontur des Querschnitts des Schafts 17 ist zwischen dem proximalen Ende 12 und dem distalen Ende 19 konstant oder im Wesentlichen konstant. Insbesondere ist die Kontur des Querschnitts des Schafts 17 kreisförmig oder elliptisch. In diesem Fall ist die in Figur 1 angedeutete Längsachse 18 des Endoskops 10 die Symmetrieachse der Mantelfläche des Schafts 17 zwischen dem proximalen Ende 12 und dem distalen Ende 19. Bei einer zylindrischen Mantelfläche des Schafts 17 ist die Längsachse 18 auch die Menge der Mittelpunkte oder Flächenschwerpunkte der Querschnitte des Schafts 17 zwischen dem proximalen Ende 12 und dem distalen Ende 19. Bei einer kreiszylindrischen Mantelfläche des Schafts 17 ist die Längsachse 18 auch die Symmetrieachse der Mantelfläche.

Am distalen Ende 19 weicht die Gestalt des Schafts 17 von der Zylindersymmetrie ab, wie dies beispielhaft in Figur 1 dargestellt ist. Insbesondere weist der Schaft 17 am distalen Ende 19 eine Öffnung auf, die durch ein in gestrichelten Linien angedeutetes transparentes Fensterbauteil 20 mit einer gewölbten Oberfläche verschlossen ist. Insbesondere verschließt das Fensterbauteil 20 die Öffnung hermetisch dicht. Die Oberfläche des Fensterbauteils 20 hat beispielsweise die Gestalt eines Ausschnitts eines Kreiszylindermantels, wobei die Symmetrieachse des Kreiszylinders senkrecht zur Längsachse 18 des Endoskops 10 und zur Zeichenebene der Figur 1 ist. Alternativ hat die Oberfläche des transparenten Fensterbauteils 20 die Gestalt eines Ausschnitts einer Kugeloberfläche oder eines rotationssymmetrischen oder nicht rotationssymmetrischen Ellipsoids.

Am distalen Ende 19 des Endoskops 10 sind im Schaft 17 optische Einrichtungen angeordnet, die in Figur 1 nicht dargestellt sind. Diese optischen Einrichtungen ermöglichen eine Variation der Blickrichtung des Endoskops zwischen einer ersten extremen Blickrichtung 21 und einer zweiten extremen Blickrichtung 22. Die Blickrichtung ist zwischen den beiden extremen Blickrichtungen 21, 22 um eine Schwenkachse 28 schwenkbar, die senkrecht zur Zeichenebene der Figur 1 ist. Die Blickrichtung ist jeweils die Richtung bezogen auf das distale Ende 19 des Endoskops 10, in der ein Gegenstand liegt, der in der Mitte eines mittels des Endoskops 10 erfassten Bilds erscheint.

Bei dem in Figur 1 dargestellten Beispiel ist die erste extreme Blickrichtung 21 parallel oder im Wesentlichen parallel zur Längsachse 18 des Endoskops 10. Zwischen den extremen Blickrichtungen 21, 22 liegt ein Winkelbereich 29, der bei dem dargestellten Beispiel ca. 120 Grad umfasst. Innerhalb dieses Winkelbereichs 29 ist die Blickrichtung des Endoskops 10 insbesondere kontinuierlich verstellbar bzw. einstellbar.

Am proximalen Ende 12 weist das Endoskop 10 ein Okular 14 oder eine Kupplung zum optischen Koppeln des Endoskops 10 mit einer Kamera auf. Ferner weist das Endoskop 10 am proximalen Ende 12 eine Kupplung 15 zum Koppeln des Endoskops 10 mit einer Lichtquelle über ein Lichtleitkabel auf. Von der Kupplung 15 führen ein oder mehrere Lichtleiter 30 durch den Schaft 14 bis zum distalen Ende 19 des Endoskops 10. Distale Enden 38 der Lichtleiter 30 sind jeweils mit einer Lichtlenkeinrichtung 40 gekoppelt. Die Lichtlenkeinrichtungen 40 sind neben dem Fensterbauteil 20, insbesondere an zwei gegenüberliegenden Rändern des Fensterbauteils 20 angeordnet.

Von einer Lichtquelle erzeugtes Beleuchtungslicht kann über ein Lichtleitkabel, die Kupplung 15 und den oder die Lichtwellenleiter 30 zum distalen Ende 19 des Endoskops 10 übertragen werden. Am distalen Ende 19 des Endoskops 10 tritt das Beleuchtungslicht durch die Lichtlenkeinrichtungen 40 hindurch aus dem Endoskop 10 aus, um zu beobachtende Gegenstände zu beleuchten. Dabei lenkt bzw. beeinflusst jede Lichtlenkeinrichtung 40 das Beleuchtungslicht hinsichtlich seiner Richtung und/oder seiner Winkelverteilung.

Die Lichtlenkeinrichtungen sind insbesondere Lichtbrechungseinrichtungen, die Beleuchtungslicht durch Brechung an einer formbaren Phasengrenzfläche steuerbar lenken. Beispiele für Lichtbrechungseinrichtungen sind nachfolgend anhand der Figuren 2 bis 7 dargestellt.

Am proximalen Ende 12 des Endoskops 10 ist eine Lichtweicheneinrichtung 70 zum steuerbaren Einkoppeln von über die Kupplung 15 empfangenem Beleuchtungslicht in einen oder mehrere der Lichtleiter 30 vorgesehen. Beispielhaft ist ein gekrümmter Spiegel 71 dargestellt, der schwenkbar ist. Alternativ kann die Lichtweicheneinrichtung 70 beispielsweise eine Lichtbrechungseinrichtung mit einer steuerbaren Grenzfläche, an der das Beleuchtungslicht steuerbar gebrochen wird, umfassen. Die Lichtweicheneinrichtung 70 ist optional. Alternativ ist das Endoskop ausgebildet, um bereitgestelltes Beleuchtungslicht jederzeit in alle Lichtleiter 30 einzukoppeln.

Ferner ist am proximalen Ende 12 des Endoskops 10 eine Steuerungseinrichtung 80 mit einem Steuerausgang 81 zum Bereitstellen eines Steuersignals vorgesehen. Der Steuerausgang 81 ist über eine oder mehrere Steuerleitungen 82 mit den Lichtbrechungseinrichtungen 40 am distalen Ende 19 des Endoskops 10 gekoppelt. Die Steuerungseinrichtung 80 ist ferner mit der Lichtweicheneinrichtung 70 gekoppelt.

Die Steuerungseinrichtung 80 ist ausgebildet, um die Lichtweicheneinrichtung 70 und über die Steuerleitungen 82 die Lichtbrechungseinrichtungen 42 abhängig von einer am Endoskop 10 eingestellten Blickrichtung 21, 22 zu steuern. Dazu ist die Steuerungseinrichtung 80 insbesondere mittelbar oder unmittelbar mit einer Benutzerschnittstelle gekoppelt, an der ein Benutzer des Endoskops 10 die Blickrichtung des Endoskops 10 einstellen kann. Alternativ oder zusätzlich kann die Steuerungseinrichtung ausgebildet sein, um die Lichtbrechungseinrichtungen 40 und die Lichtweicheneinrichtung 70 abhängig von einer am Endoskop 10 eingestellten Brennweite bzw. einem eingestellten Bildfeldwinkel zu steuern. Die Steuerungseinrichtung 80 ist ausgebildet, um die Lichtbrechungseinrichtungen 40 und die Lichtweicheneinrichtung 70 so zu steuern, dass jederzeit ein möglichst hoher Anteil bereitgestellten Beleuchtungslichts in den momentan mittels des Endoskops 10 beobachteten bzw. optisch erfassten Raumbereichs abgestrahlt und möglichst wenig Beleuchtungslicht in momentan nicht beobachtete bzw. nicht optisch erfasste Bereiche abgestrahlt wird.

Die Figuren 2 bis 5 zeigen schematische Schnittdarstellungen eines Ausführungsbeispiels einer Lichtbrechungseinrichtung 40. Die dargestellte Schnittebene ist parallel zur Zeichenebene der Figur 1. Jeweils in gestrichelter Linie ist die gekrümmte Kontur des distalen Endes 19 des Endoskops 10 angedeutet.

Die Lichtbrechungseinrichtung 40 umfasst ein erstes Fensterbauteil 42 an einer Lichteintrittsseite und ein zweites Fensterbauteil 48 an einer gegenüberliegenden Lichtaustrittsseite. Das erste Fensterbauteil 42 und das zweite Fensterbauteil 48 weisen jeweils ein für das vorgesehene Beleuchtungslicht transparentes Material auf. Das erste Fensterbauteil 42 und das zweite Fensterbauteil 48 können jeweils parallele und ebene oder - abweichend von den Darstellungen in den Figuren 2 bis 5 - eine oder zwei gekrümmte Oberflächen aufweisen. Von den Darstellungen in den Figuren 2 bis 5 abweichend gekrümmte Oberflächen der Fensterbauteile 42, 48 können mit einer sammelnden oder aufweitenden Wirkung zur Strahlformung beitragen.

Das erste Fensterbauteil 42 und das zweite Fensterbauteil 48 sind durch eine ringförmige Wand 43 miteinander verbunden. Die Wand 43 weist insbesondere die Gestalt eines Zylinders mit kreisförmigem, elliptischem, quadratischem, rechteckigem oder anderem Querschnitt auf. Die Wand 43 kann das gleiche Material aufweisen wie die Fensterbauteile 42, 48. Alternativ kann die Wand 43 ein anderes Material, insbesondere ein nichttransparentes Material aufweisen.

Die Wand 43, das erste Fensterbauteil 42 und das zweite Fensterbauteil 48 sind gefügt, insbesondere verklebt oder verschweißt, um einen Hohlraum hermetisch dicht zu umschließen. In dem durch die Fensterbauteile 42, 48 und die Wand 43 umschlossenen Hohlraum sind zwei nicht mischbare Fluide, insbesondere eine erste Flüssigkeit 44 und eine zweite Flüssigkeit 46 angeordnet. Die erste Flüssigkeit 44 und die zweite Flüssigkeit 46 sind nicht oder im Wesentlichen nicht mischbar und bilden eine Phasengrenzfläche 45. Beispielsweise sind die erste Flüssigkeit 44 eine wässrige Salzlösung und die zweite Flüssigkeit 46 ein Öl oder umgekehrt. Beide Flüssigkeiten 44, 46 sind für das vorgesehene Beleuchtungslicht transparent und weisen möglichst unterschiedlichste Brechungsindizes auf. Beide Flüssigkeiten 44, 46 weisen ferner hinsichtlich ihrer Wechselwirkung mit elektrostatischen Feldern unterschiedliche Eigenschaften auf, insbesondere unterschiedliche elektrische Permittivitäten bzw. unterschiedliche Dielektrizitätszahlen.

Am ersten Fensterbauteil 42 und an der Wand 43 sind mehrere Elektroden 51, 52, 53, 54 zum Erzeugen elektrischer Felder vorgesehen. Jede einzelne Elektrode 51, 52, 53, 54 ist über eine Steuerleitung 82 mit der Steuerungseinrichtung 80 (vgl. Figur 1) gekoppelt. Jede Elektrode 51, 52, 53, 54 kann insbesondere - wie in den Figuren 2 bis 5 angedeutet - an einer äußeren Oberfläche des Fensterbauteils 42 oder der Wand 43 oder - abweichend von den Darstellungen in den Figuren 2 bis 5 - an einer inneren Oberflächen des Fensterbauteils 42 oder der Wand 43 angeordnet sein. Abweichend von den Darstellungen in den Figuren 2 bis 5 können ferner eine oder mehrere transparente Elektroden am Fensterbauteil 48 vorgesehen sein.

Die Lichtbrechungseinrichtung 40 ist mit dem distalen Ende 38 des Lichtleiters 30 gekoppelt. Insbesondere ist die Lichtaustrittsfläche 39 am distalen Ende 38 des Lichtleiters 30 mit der durch das erste Fensterbauteil 42 der Lichtbrechungseinrichtung 40 gebildeten Lichteintrittsfläche 41 verklebt, verkittet oder verschweißt, so dass die Lichtaustrittsfläche 39 des Lichteleiters 30 gleichzeitig die Lichteintrittsfläche 41 der Lichtbrechungseinrichtung 40 ist. Zur Minderung von Reflexionsverlusten können an der Grenzfläche zwischen Lichtleiter 30 und Lichtbrechungseinrichtung 40 eine oder mehrere Schichten vorgesehen sein, die Reflexionen mindern. Die der Lichteintrittsfläche 41 gegenüberliegende und durch das zweite Fensterbauteil 48 gebildete Lichtaustrittsfläche 49 der Lichtbrechungseinrichtung 40 ist insbesondere bündig oder im Wesentlichen bündig mit der in den Figuren 2 bis 5 durch gestrichelte Linien angedeuteten äußeren Oberfläche des distalen Endes des Endoskops angeordnet.

Die vorgesehene Ausbreitungsrichtung 64 von Beleuchtungslicht in der Lichtbrechungseinrichtung 40 ist die Richtung vom ersten Fensterbauteil 42 zum zweiten Fensterbauteil 48.

Mittels des Lichtleiters 30 kann Beleuchtungslicht vom proximalen Ende 12 zum distalen Ende 19 des Endoskops 10 (vgl. Figur 1) übertragen und dort in die Lichtbrechungseinrichtung 40 eingekoppelt werden. Das Beleuchtungslicht wird aufgrund der unterschiedlichen Brechungsindizes der Flüssigkeiten 44, 46 und der Fensterbauteile 42, 49 mehrfach gebrochen, insbesondere an der Phasengrenzfläche 45 zwischen den Flüssigkeiten 44, 46, bevor es durch die Lichtaustrittsfläche 49 aus der Lichtbrechungseinrichtung 40 und aus dem Endoskop austritt, um zu beobachtende Gegenstände zu beleuchten.

Durch Anlegen unterschiedlicher elektrischer Potenziale an die Elektroden 51, 52, 53, 54 kann ein elektrisches Feld in der Lichtbrechungseinrichtung 40 erzeugt werden. Aufgrund unterschiedlicher Wechselwirkung der Flüssigkeiten 44, 46 mit einem elektrischen Feld beeinflusst dieses die Gestalt der Phasengrenzfläche 45 zwischen den Flüssigkeiten 44, 46. Durch Variation der über die Steuerleitungen 82 an die Elektroden 51, 52, 53, 54 angelegten elektrischen Potenziale kann die räumliche Gestalt der Phasengrenzfläche 45 zwischen den Flüssigkeiten 44, 46 variiert werden.

In den Figuren 2 bis 5 sind beispielhaft vier verschiedene Formen bzw. Gestalten der Phasengrenzfläche 45 zwischen den Flüssigkeiten 44, 46 gezeigt, die durch Anlegen unterschiedlicher elektrischer Potenziale an die Elektroden 51, 52, 53, 54 erzeugt werden können. Zum Erzeugen der dargestellten Formen der Phasengrenzfläche 45 können andere als die dargestellte Anordnung von Elektroden vorteilhaft sein.

Bei den in den Figuren 2 und 3 gezeigten Beispielen ist die Phasengrenzfläche 45 zwischen den Flüssigkeiten 44, 46 jeweils eben, aber in unterschiedliche Richtungen gekippt. Die unterschiedliche Brechung des Beleuchtungslichts an der Phasengrenzfläche 45 bewirkt zwei verschiedene Beleuchtungsrichtungen 61, 62, in die das Beleuchtungslicht abgestrahlt wird. Durch eine kontinuierliche Variation der an die Elektroden 51, 52, 53, 54 angelegten elektrischen Potenziale kann die Beleuchtungsrichtung zumindest zwischen den in den Figuren 2 und 3 angedeuteten Beleuchtungsrichtungen 61, 62 kontinuierlich variiert werden.

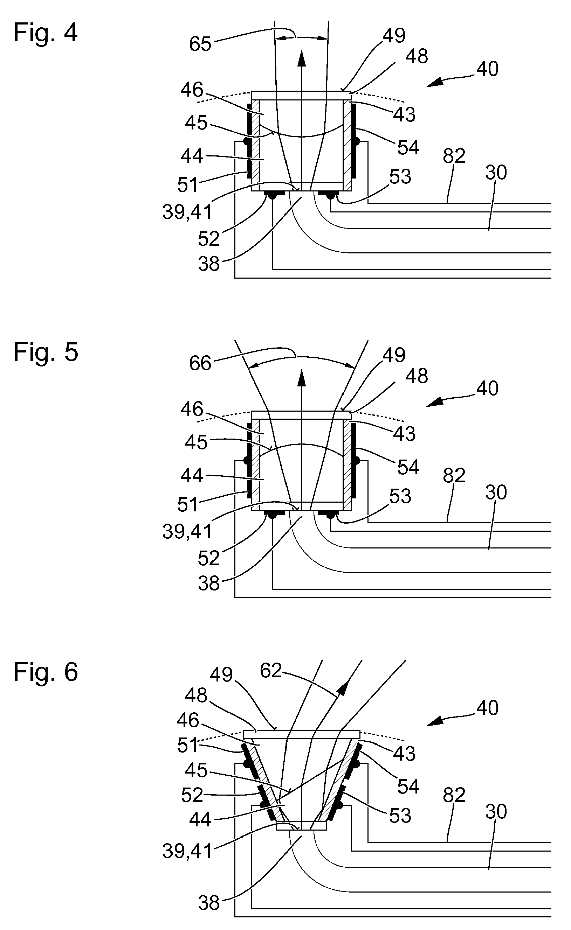

Bei den in den Figuren 4 und 5 dargestellten Beispielen ist die Phasengrenzfläche 45 zwischen den Flüssigkeiten 44, 46 jeweils gewölbt, und zwar in entgegengesetzte Richtungen. Die resultierende unterschiedliche Brechung des Beleuchtungslichts bewirkt unterschiedliche Öffnungswinkel 65, 66 des an der Lichtaustrittsfläche 49 der Lichtbrechungseinrichtung 40 aus dem Endoskop austretenden Lichtbündels. Durch kontinuierliche Variation der an die Elektroden 51, 52, 53, 54 angelegten elektrischen Potenziale kann der Öffnungswinkel des von der Lichtbrechungseinrichtung 40 geformten Lichtbündels zumindest zwischen den in den Figuren 4 und 5 gezeigten Öffnungswinkeln 65, 66 kontinuierlich variiert werden.

Während in den Figuren 2 bis 5 die Phasengrenzfläche 45 zwischen den Flüssigkeiten 44, 46 nur entweder eben und gekippt oder symmetrisch und gekrümmt gezeigt ist, kann sie durch Anlegen entsprechender elektrischer Potenziale an die Elektroden 51, 52, 53, 54 auch gleichzeitig gekippt und gekrümmt werden. Statt Elektroden oder zusätzlich zu diesen können abweichend von den Darstellungen in den Figuren 2 bis 5 eine oder mehrere Spulen zum Erzeugen eines Magnetfelds vorgesehen sein. In diesem Fall weisen die Flüssigkeiten 44, 46 unterschiedliche Wechselwirkungen mit einem Magnetfeld, insbesondere unterschiedliche magnetische Permeabilitäten bzw. unterschiedliche magnetische Suszeptibilitäten auf.

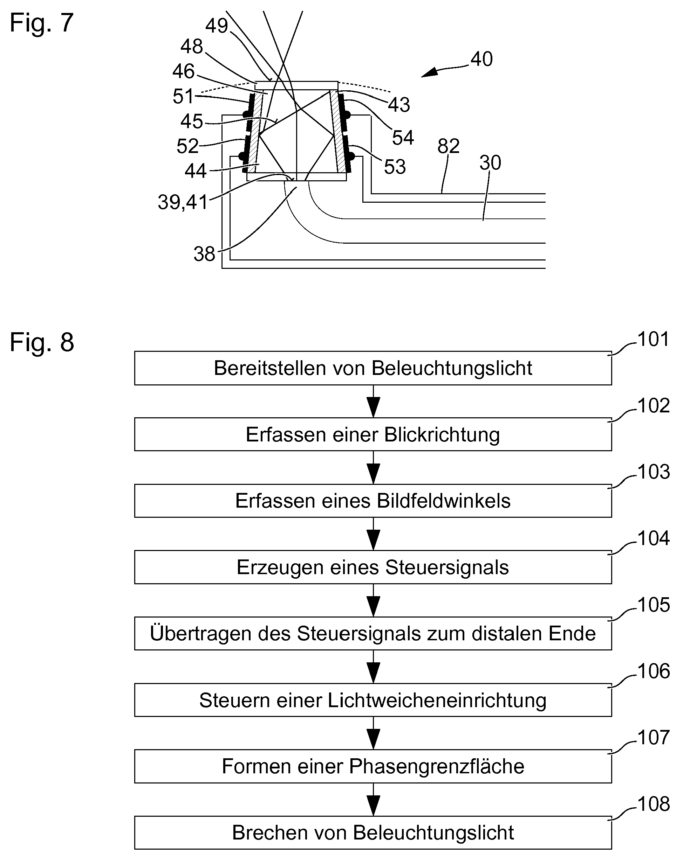

Die Figuren 6 und 7 zeigen schematische Darstellungen weiterer Lichtbrechungseinrichtungen 40, die der oben anhand der Figuren 2 bis 5 dargestellten Lichtbrechungseinrichtung in einigen Merkmalen ähneln. Die in den Figuren 6 und 7 dargestellten Lichtbrechungseinrichtungen 40 unterscheiden sich von der oben anhand der Figuren 2 bis 5 dargestellten Lichtbrechungseinrichtung insbesondere durch eine andere beispielhafte Anordnung der Elektroden 51, 52, 53, 54. Bei allen in den Figuren 2 bis 7 dargestellten Lichtbrechungseinrichtungen können weniger oder mehr Elektroden als dargestellt vorgesehen sein. Ferner können die Elektroden anders als in den Figuren 2 bis 7 angedeutet angeordnet sein.

Die Lichtbrechungseinrichtungen der Figuren 6 und 7 unterscheiden sich von der in den Figuren 2 bis 5 dargestellten Lichtbrechungseinrichtung ferner durch eine nichtzylindrische Gestalt der Wand 43 zwischen dem ersten Fensterbauteil 42 und dem zweiten Fensterbauteil 48. Bei den in den Figuren 6 und 7 gezeigten Lichtbrechungseinrichtungen 40 weist die Wand 43 jeweils die Gestalt eines Ausschnitts einer Mantelfläche einer Pyramide mit quadratischer, rechteckiger oder polygonaler Grundfläche oder eines Kegels mit kreisförmiger, elliptischer oder anderer Grundfläche auf. Ferner können in den Figuren 6 und 7 nicht angeschnittene Bereiche der Wand 43 eine andere Gestalt aufweisen, beispielsweise eben und parallel zueinander und zu den Schnittebenen der Figuren 6 und 7 sein.

Der in der vorgesehenen Ausbreitungsrichtung von Beleuchtungslicht variierende Querschnitt der Lichtbrechungseinrichtung 40 beeinflusst die Divergenz bzw. die Winkelverteilung des an der Lichtaustrittsfläche 49 der Lichtbrechungseinrichtung 40 aus dem Endoskop austretenden Beleuchtungslichts. Insbesondere kann ein in Ausbreitungsrichtung des Beleuchtungslichts sich verbreiternder Querschnitt, wie er in Figur 6 angedeutet ist, die Divergenz des austretenden Lichts vermindern. Eine in der vorgesehenen Ausbreitungsrichtung von Beleuchtungslicht abnehmende Breite des Querschnitts der Lichtbrechungseinrichtung 40, wie sie in Figur 7 angedeutet ist, kann die Divergenz des Beleuchtungslichts vergrößern.

Figur 8 zeigt ein schematisches Flussdiagramm eines Verfahrens zum Beleuchten eines einstellbaren Sichtfelds eines Endoskops. Obwohl das Verfahren auch mit einem Endoskop ausführbar ist, das andere als die oben anhand der Figuren 1 bis 7 dargestellten Merkmale aufweist, werden nachfolgend beispielhaft Bezugszeichen aus den Figuren 1 bis 7 verwendet, um das Verständnis zu vereinfachen. Bei einem ersten Schritt 101 wird Beleuchtungslicht bereitgestellt. Das Beleuchtungslicht kann am distalen Ende 19, am proximalen Ende 12 des Endoskops oder in einer separaten Lichtquelleneinrichtung erzeugt und mittels eines Lichtleitkabels zum Endoskop 10 übertragen werden. Erforderlichenfalls wird das Beleuchtungslicht vom proximalen Ende 12 zum distalen Ende 19 des Endoskops mittels eines Lichtleiters 30 übertragen.

Bei einem zweiten Schritt 102 wird eine durch Benutzer eingestellte momentane Blickrichtung 21, 22 des Endoskops 10 erfasst. Bei einem dritten Schritt 103 wird ein durch Benutzer an einer Benutzerschnittstelle des Endoskops 10 momentan eingestellter Bildfeldwinkel erfasst. Bei einem vierten Schritt 104 wird, insbesondere durch eine Steuerungseinrichtung 80, abhängig von der erfassten Blickrichtung und dem erfassten Bildfeldwinkel ein Steuersignal erzeugt. Das Steuersignal ist beispielsweise ein elektrisches Spannung- oder Stromsignal. Bei einem fünften Schritt 105 wird das beim vierten Schritt 104 erzeugte Steuersignal zum distalen Ende 19 des Endoskops übertragen.

Bei einem optionalen sechsten Schritt 106 wird eine Lichtweicheneinrichtung 70 abhängig von der beim zweiten Schritt 102 erfassten Blickrichtung und von dem beim dritten Schritt erfassten Bildfeldwinkel gesteuert, um beim ersten Schritt 101 bereitgestelltes Beleuchtungslicht in einen oder mehrere Lichtleiter 30 von einer Mehrzahl von Lichtleitern 30 einzukoppeln.

Bei einem siebten Schritt 107 wird gesteuert durch das beim vierten Schritt 104 erzeugte und beim fünften Schritt 105 zum distalen Ende 19 des Endoskops 10 übertragene Steuersignal eine Phasengrenzfläche zwischen zwei Fluiden mit unterschiedlichen optischen Eigenschaften geformt, insbesondere gekippt und/oder gekrümmt. Bei einem achten Schritt 108 wird das beim ersten Schritt 101 bereitgestellte und erforderlichenfalls zum distalen Ende 19 des Endoskops 10 übertragene Beleuchtungslicht an der beim siebten Schritt 107 geformten Phasengrenzfläche gebrochen. Die Brechung des Beleuchtungslichts und damit seine Richtung und Winkelverteilung sind von der beim siebten Schritt 107 erzeugten Form bzw. Gestalt der Phasengrenzfläche 45 abhängig.

### Bezugszeichen

- 10: Endoskop
- 12: proximales Ende des Endoskops 10
- 14: Okular
- 15: Kupplung für Lichtleitkabel am proximalen Ende 12 des Endoskops 10
- 17: Schaft des Endoskops 10
- 18: Längsachse des Endoskops 10
- 19: distales Ende des Endoskops 10
- 20: Fensterbauteil
- 21: erste extreme Blickrichtung
- 22: zweite extreme Blickrichtung
- 28: Schwenkachse der Blickrichtung 21, 22
- 29: Winkelbereich zwischen den extremen Blickrichtungen 21, 22
- 30: Lichtleiter
- 31: Lichteintrittsfläche des Lichtleiters 30
- 32: proximales Ende des Lichtleiters 30
- 38: distales Ende des Lichtleiters 30
- 39: Lichtaustrittsfläche des Lichtleiters 30
- 40: Lichtbrechungseinrichtung
- 41: Lichteintrittsfläche der Lichtbrechungseinrichtung 40
- 42: Fensterbauteil der Lichtbrechungseinrichtung 40
- 43: Wand der Lichtbrechungseinrichtung 40
- 44: erste Flüssigkeit in der Lichtbrechungseinrichtung 40
- 45: Phasengrenzfläche zwischen der ersten Flüssigkeit 44 und der zweiten Flüssigkeit 46
- 46: zweite Flüssigkeit in der Lichtbrechungseinrichtung 40
- 48: Fensterbauteil der Lichtbrechungseinrichtung 40
- 49: Lichtaustrittsfläche der Lichtbrechungseinrichtung 40
- 51: erste Elektrode an der Lichtbrechungseinrichtung 40
- 52: zweite Elektrode an der Lichtbrechungseinrichtung 40
- 53: dritte Elektrode an der Lichtbrechungseinrichtung 40
- 54: vierte Elektrode an der Lichtbrechungseinrichtung 40
- 61: erste Beleuchtungsrichtung
- 62: zweite Beleuchtungsrichtung
- 64: vorgesehene Ausbreitungsrichtung von Beleuchtungslicht in der Lichtbrechungseinrichtung 40
- 65: erster Öffnungswinkel
- 66: zweiter Öffnungswinkel
- 70: Lichtweicheneinrichtung
- 71: Spiegel in der Lichtweicheneinrichtung 70
- 80: Steuerungseinrichtung zum Steuern der Lichtbrechungseinrichtung 40
- 81: Steuerausgang zum Bereitstellen eines Steuersignal für die Lichtbrechungseinrichtung 40
- 82: Steuerleitung zum Übertragen eines Steuersignals von der Steuerungseinrichtung 80 zu der Lichtbrechungseinrichtung 40
- 101: erster Schritt (Bereitstellen von Beleuchtungslicht)
- 102: zweiter Schritt (Erfassen einer Blickrichtung)
- 103: dritter Schritt (Erfassen eines Bildfeldwinkels)
- 104: vierter Schritt (Erzeugen eines Steuersignals)
- 105: fünfter Schritt (Übertragen des Steuersignals zum distalen Ende)
- 106: sechster Schritt (Steuern einer Lichtweicheneinrichtung)
- 107: siebter Schritt (Formen einer Phasengrenzfläche)
- 108: achter Schritt (Brechen von Beleuchtungslicht)

## Patentansprüche

1. **Endoskop** (10), mit:
einem proximalen Ende (12), einem distalen Ende (19) und einem starren, flexiblen oder
teilweise flexiblen Schaft (17) der sich vom proximalen Ende (12) bis zum distalen Ende (19) erstreckt,
einer **Lichtaustrittsfläche** (49) am distalen Ende (19) des Endoskops (10);
wobei zumindest entweder eine Blickrichtung (21, 22) oder ein Bildfeldwinkel (25, 26) des Endoskops (10) einstellbar ist, ferner mit:
einer steuerbaren Lichtbrechungseinrichtung (40) zum steuerbaren Brechen von Beleuchtungslicht mittels einer formbaren **Phasengrenzfläche** (45) **zwischen zwei Fluiden** (44, 46),
wobei die steuerbare Lichtbrechungseinrichtung (40) am distalen Ende (19) des Endoskops (10) angeordnet ist,wobei die Lichtaustrittsfläche (49) am distalen Ende (19) des Endoskops (10) eine **Lichtaustrittsfläche der steuerbaren Lichtbrechungseinrichtung** (40) ist,
einer **Steuerungseinrichtung** (80) zum Erzeugen eines Steuersignals zum Steuern der Lichtbrechungseinrichtung (40), wobei die Steuerungseinrichtung (80) ausgebildet ist, um die Lichtbrechungseinrichtung (40) zumindest entweder abhängig von der eingestellten Blickrichtung (21, 22) oder abhängig von dem eingestellten Bildfeldwinkel (25, 26) zu steuern.

2. Endoskop (10) nach einem der vorangehenden Ansprüche, wobei
das Endoskop (10) eine Mehrzahl von Lichtaustrittsflächen (49) am distalen Ende (19) aufweist,
an jeder der Mehrzahl von Lichtaustrittsfläche (49) eine Lichtbrechungseinrichtung (40) angeordnet ist.

3. Endoskop (10) nach einem der vorangehenden Ansprüche, bei der die steuerbare Lichtbrechungseinrichtung (40) zumindest entweder zum Einstellen der **Beleuchtungsrichtung** (61, 62) oder zum Einstellen des **Öffnungswinkels** (67, 68) des zu beleuchtenden Raumbereichs ausgebildet ist.

4. Endoskop (10) nach einem der vorangehenden Ansprüche, bei dem die Lichtbrechungseinrichtung (40) einen **Querschnitt** aufweist, zumindest entweder dessen **Fläche** oder dessen **Breite** in der vorgesehenen Ausbreitungsrichtung (64) von Beleuchtungslicht zu**nimmt.**

5. Endoskop (10) nach einem der Ansprüche 1 bis 3, bei dem die Lichtbrechungseinrichtung (40) einen **Querschnitt** () aufweist, zumindest entweder dessen **Fläche** oder dessen **Brei**te (69) in der vorgesehenen Ausbreitungsrichtung (64) von Beleuchtungslicht **abnimmt.**

6. Endoskop (10) nach einem der vorangehenden Ansprüche, ferner mit:
einer Felderzeugungseinrichtung (51, 54, 56, 59) zum Erzeugen eines elektrischen oder magnetischen Felds zum Formen der Phasengrenzfläche (45).

7. Endoskop (10) nach dem vorangehenden Anspruch, wobei die Steuerungseinrichtung (80) ausgebildet ist, um für mehrere Lichtbrechungseinrichtungen (40) je ein Steuersignal zu erzeugen.

8. Endoskop (10) nach einem der vorangehenden Ansprüche, ferner mit:
einem **Lichtleiter** (30) zum Übertragen von Beleuchtungslicht zum distalen Ende (19) des Endoskops (10), mit einer Lichtaustrittsfläche (39), die am distalen Ende (19) des Endoskops (10) angeordnet ist,
wobei die Lichtbrechungseinrichtung (40) zwischen der Lichtaustrittsfläche (39) des Lichtleiters (20) und der Lichtaustrittsfläche (49) des Endoskops (10) angeordnet ist.

9. Endoskop (10) nach dem vorangehenden Anspruch, ferner mit:
einer Mehrzahl von **Lichtleitern** (30) zum Übertragen von Beleuchtungslicht zum distalen Ende (19) des Endoskops (10), mit je einer Lichtaustrittsfläche (39), die am distalen Ende (19) des Endoskops (10) angeordnet ist,
einer **Lichtweicheneinrichtung** (70) zum steuerbaren Einkoppeln von Beleuchtungslicht in einen oder mehrere der Mehrzahl von Lichtleitern (30)

10. **Verfahren zum Beleuchten** eines einstellbaren Sichtfelds (21, 22, 25, 26) eines Endoskops (10) für technische Anwendungen mit einem proximalen Ende 12, einem distalen Ende 19 und einem starren,
flexiblen oder teilweise flexiblen Schaft 17, der sich vom proximalen Ende 12 bis zum distalen Ende 19 erstreckt, mit folgenden Schritten:
Bereitstellen (101) von **Beleuchtungslicht;**
Formen (106) einer **Phasengrenzfläche** (45) **zwischen zwei Fluiden** (44, 46) am distalen Ende (19) des Endoskops (10) zum steuerbaren Brechen von Beleuchtungslicht,
wobei die Phasengrenzfläche (45) zumindest entweder abhängig von der momentan eingestellten Blickrichtung oder abhängig von dem eingestellten Bildfeldwinkel (21, 22, 25, 26) des Endoskops (10) geformt wird,
Erzeugen (104) eines **Steuersignals;**
Übertragen (105) des Steuersignals **zum distalen Ende** (19) des Endoskops (10),
wobei das Formen (106) der Phasengrenzfläche (45) am distalen Ende (19) des Endoskops (10) mittels des Steuersignals zumindest entweder abhängig von der eingestellten Blickrichtung oder abhängig von dem eingestellten Bildfeldwinkel gesteuert wird.

11. Verfahren nach Anspruch 10, wobei mehrere Steuersignale erzeugt (104) und zum distalen Ende (19) des Endoskops (10) übertragen (105) werden, um zum Brechen (107) von Beleuchtungslicht das Formen (106) mehrerer Phasengrenzflächen (45) am distalen Ende (19) des Endoskops (10) durch je ein Steuersignal zu steuern.

## Claims

1. Endoscope (10), comprising:
a proximal end (12), a distal end (19) and a rigid, flexible or partly flexible shank (17) which extends from the proximal end (12) to the distal end (19),
a light-exit face (49) at the distal end (19) of the endoscope (10);
wherein at least either a viewing direction (21, 22) or an image field angle (25, 26) of the endoscope (10) is adjustable, furthermore comprising:
a controllable light-refraction device (40) for controllably refracting illumination light by means of a formable phase interface (45) between two fluids (44, 46),
wherein the controllable light-refraction device (40) is arranged at the distal end (19) of the endoscope (10), wherein the light-exit face (49) at the distal end (19) of the endoscope (10) is a light-exit face of the controllable light-refraction device (40),
a control device (80) for generating a control signal for controlling the light-refraction device (40), wherein the control device (80) is embodied to control the light-refraction device (40) at least either in a manner dependent on the adjusted viewing direction (21, 22) or in a manner dependent on the adjusted image field angle (25, 26) .

2. Endoscope (10) according to one of the preceding claims, wherein
the endoscope (10) has a plurality of light-exit faces (49) at the distal end (19),
a light-refraction apparatus (40) is arranged on each one of the plurality of light-exit faces (49).

3. Endoscope (10) according to one of the preceding claims, wherein the controllable light-refraction device (40) is embodied at least either to adjust the illumination direction (61, 62) or to adjust the aperture angle (67, 68) of the spatial region to be illuminated.

4. Endoscope (10) according to one of the preceding claims, wherein the light-refraction device (40) has a cross section, at least either the area thereof or the width thereof increasing in the propagation direction (64) of illumination light provided.

5. Endoscope (10) according to one of Claims 1 to 3, wherein the light-refraction device (40) has a cross section (), at least either the area thereof or the width (69) thereof decreasing in the propagation direction (64) of illumination light provided.

6. Endoscope (10) according to one of the preceding claims, furthermore comprising:
a field generating device (51, 54, 56, 59) for generating an electric or magnetic field for forming the phase interface (45).

7. Endoscope (10) according to the preceding claim, wherein the control device (80) is embodied to generate respectively one control signal for a plurality of light-refraction devices (40).

8. Endoscope (10) according to one of the preceding claims, furthermore comprising:
a light guide (30) for transmitting illumination light to the distal end (19) of the endoscope (10), comprising a light-exit face (39) which is arranged at the distal end (19) of the endoscope (10),
wherein the light-refraction device (40) is arranged between the light-exit face (39) of the light guide (20) and the light-exit face (49) of the endoscope (10).

9. Endoscope (10) according to the preceding claim, furthermore comprising:
a plurality of light guides (30) for transmitting illumination light to the distal end (19) of the endoscope (10), each comprising a light-exit face (39) which is arranged at the distal end (19) of the endoscope (10),
a light-switching device (70) for controllably coupling illumination light into one or more of the plurality of light guides (30).

10. Method for illuminating an adjustable field of view (21, 22, 25, 26) of an endoscope (10) for technical applications, said endoscope comprising a proximal end 12, a distal end 19 and a rigid, flexible or partly flexible shank 17, which extends from the proximal end 12 to the distal end 19, comprising the following steps:
providing (101) illumination light;
forming (106) a phase interface (45) between two fluids (44, 46) at the distal end (19) of the endoscope (10) for controllably refracting illumination light,
wherein the phase interface (45) is formed at least either dependent on the currently adjusted viewing direction or dependent on the adjusted image field angle (21, 22, 25, 26) of the endoscope (10),
generating (104) a control signal;
transmitting (105) the control signal to the distal end (19) of the endoscope (10), wherein the forming (106) of the phase interface (45) at the distal end (19) of the endoscope (10) is controlled by means of the control signal, at least either dependent on the adjusted viewing direction or dependent on the adjusted image field angle.

11. Method according to Claim 10, wherein a plurality of control signals are generated (104) and transmitted (105) to the distal end (19) of the endoscope (10) in order, for the purposes of refracting (107) illumination light, to control the formation (106) of a plurality of phase interfaces (45) at the distal end (19) of the endoscope (10) by a control signal in each case.

## Revendications

1. Endoscope (10), comportant :
une extrémité proximale (12), une extrémité distale (19) et une tige (17) rigide, flexible ou partiellement flexible qui s'étend de l'extrémité proximale (12) à l'extrémité distale (19),
une surface de sortie de lumière (49) à l'extrémité distale (19) de l'endoscope (10) ;
dans lequel au moins soit une direction d'observation (21, 22), soit un angle de champ d'image (25, 26) de l'endoscope (10) sont réglables, comportant en outre :
un dispositif de réfraction lumineuse (40) pour réfracter d'une manière pouvant être commandée la lumière d'éclairement au moyen d'une interface entre phases déformable (45) entre deux fluides (44, 46),
dans lequel le dispositif de réfraction lumineuse pouvant être commandé (40) est disposé à l'extrémité distale (19) de l'endoscope (10), dans lequel la surface de sortie de lumière (49) à l'extrémité distale (19) de l'endoscope (10) est une surface de sortie de lumière du dispositif de réfraction lumineuse pouvant être commandé (40),
un dispositif de commande (80) destiné à générer un signal de commande pour commander le dispositif de réfraction lumineuse (40), dans lequel le dispositif de commande (80) est conçu pour commander le dispositif de réfraction lumineuse (40) au moins soit en fonction de la direction d'observation réglée (21, 22), soit en fonction de l'angle de champ d'image réglé (25, 26).

2. Endoscope (10) selon l'une quelconque des revendications précédentes, dans lequel
l'endoscope (10) comporte une pluralité de surfaces de sortie de lumière (49) à l'extrémité distale (19),
un dispositif de réfraction lumineuse (40) est disposé sur chacune de la pluralité de surfaces de sortie de lumière (49).

3. Endoscope (10) selon l'une quelconque des revendications précédentes, dans lequel le dispositif de réfraction lumineuse pouvant être commandé (40) est conçu au moins soit pour régler la direction d'éclairement (61, 62), soit pour régler l'angle d'ouverture (67, 68) de la région spatiale à éclairer.

4. Endoscope (10) selon l'une quelconque des revendications précédentes, dans lequel le dispositif de réfraction lumineuse (40) présente une section transversale dont au moins soit la surface, soit la largeur augmente dans la direction de propagation prévue (64) de la lumière d'éclairement.

5. Endoscope (10) selon l'une quelconque des revendications 1 à 3, dans lequel le dispositif de réfraction lumineuse (40) présente une section transversale () dont au moins soit la surface, soit la largeur (69) diminue dans la direction de propagation prévue (64) de la lumière d'éclairement.

6. Endoscope (10) selon l'une quelconque des revendications précédentes, comportant en outre :
un dispositif générateur de champ (51, 54, 56, 59) destiné à générer un champ électrique ou magnétique pour déformer l'interface entre phases (45).

7. Endoscope (10) selon la revendication précédente, dans lequel le dispositif de commande (80) est conçu pour générer un signal de commande respectif pour plusieurs dispositifs de réfraction lumineuse (40) .

8. Endoscope (10) selon l'une quelconque des revendications précédentes, comportant en outre :
un guide optique (30) destiné à transmettre une lumière d'éclairement vers l'extrémité distale (19) de l'endoscope (10), ayant une surface de sortie de lumière (39) qui est disposée à l'extrémité distale (19) de l'endoscope (10),
dans lequel le dispositif de réfraction lumineuse (40) est disposé entre la surface de sortie de lumière (39) du guide optique (20) et la surface de sortie de lumière (49) de l'endoscope (10).

9. Endoscope (10) selon la revendication précédente, comportant en outre :
une pluralité de guides optiques (30) destinés à transporter une lumière d'éclairement vers l'extrémité distale (19) de l'endoscope (10), comportant chacun une surface de sortie de lumière (39) qui est disposée à l'extrémité distale (19) de l'endoscope (10),
un dispositif de répartition de lumière (70) destiné à injecter d'une manière pouvant être commandée une lumière d'éclairement dans u ou plusieurs de la pluralité de guides optiques (30).

10. Procédé d'éclairement d'un champ visuel réglable (21, 22, 25, 26) d'un endoscope (10) destiné à des applications techniques, comportant une extrémité proximale (12), une extrémité distale (19) et une tige (17) rigide, flexible ou partiellement flexible, qui s'étend de l'extrémité proximale (12) à l'extrémité distale (19), comportant les étapes consistant à :
fournir (101) une lumière d'éclairement ;
déformer (106) une interface entre phases (45) entre deux fluides (44, 46) à l'extrémité distale (19) de l'endoscope (10) pour réfracter d'une manière pouvant être commandée la lumière d'éclairement,
dans lequel l'interface entre phases (45) est déformée au moins soit en fonction de la direction d'observation réglée à l'instant courant, soit en fonction de l'angle de champ d'image réglé (21, 22, 25, 26) de l'endoscope (10),
générer (104) un signal de commande ;
transmettre (105) le signal de commande vers l'extrémité distale (19) de l'endoscope (10),
dans lequel la déformation (106) de l'interface entre phases (45) est commandée à l'extrémité distale (19) de l'endoscope (10) au moyen du signal de commande au moins soit en fonction de la direction d'observation réglée, soit en fonction de l'angle de champ d'image réglé.

11. Procédé selon la revendication 10, dans lequel plusieurs signaux de commande sont générés (104) et sont transmis (105) à l'extrémité distale (19) de l'endoscope (10) pour commander, afin de réfracter (107) la lumière d'éclairement, la déformation (106) de plusieurs interfaces entre phases (45) à l'extrémité distale (19) de l'endoscope (10) au moyen d'un signal de commande respectif.
